# EUROPEAN PATENT APPLICATION

(11) **EP 1 994 955 A1**
(43) Date of publication of application: **26.11.2008**
(21) Application number: 07737618.4
(22) Date of filing: 01.03.2007
(51) Int. Cl.: A61M 35/00, A61B 17/00, A61B 17/11, B05B 7/08

(54) **APPLICATOR**

(30) Priority: 13.03.2006 JP 2006068315
(71) Applicant: TERUMO KABUSHIKI KAISHA, Tokyo 151-0072 (JP)
(72) Inventor: YOKOYAMA, Kenji, Ashigarakamigun, Kanagawa 259-0151 (JP)
(74) Representative: Dossmann, Gérard
(86) International application number: PCT/JP2007/053938
(87) International publication number: WO 2007/105496

(57) **Abstract**

An applicator (1) has syringe outer tubes (21) having mouths formed projected at the forward ends of the syringe outer tubes, gaskets (24) inserted in the syringe outer tubes, and pushers (26) for moving the gaskets along the longitudinal direction of the syringe outer tubes. To use the applicator (1), a first syringe (2) and a second syringe (3) that are filled with liquid are placed in spaces formed by the syringe outer tubes (21) and the gaskets (24). The applicator (1) has an applicator body (7) where the first syringe (2) and the second syringe (3) arranged side by side and fixed, a gas flow path connected to gas supply means (300) for supplying gas and through which the gas from the gas supply means (300) passes, a nozzle (4) for discharging liquid having passed the mouth of the first syringe (2) and liquid having passed the mouth of the second syringe (3), an operation section (8) for pressing both the pusher of the first syringe and the pusher of the second syringe toward the forward ends, and open/close means (9) provided at the operation section (8) and closing and opening the gas flow path. The open/close means (9) operates to open the gas flow path in operative association with pressing operation by the operation section (8).

## Description

### TECHNICAL FIELD

The present invention relates to an applicator.

### BACKGROUND ART

Conventionally, there is known a method in which two or more liquids are mixed and sprayed to the affected part or the like to form an adhesion preventive material, a biological tissue adhesive, or the like. Thus, an applicator therefor has been under development.

Such an applicator is configured to feed components which coagulate upon mixing, such as a thrombin-containing solution and a fibrinogen-containing solution, in a mutually separated manner to the vicinity of the affected part, and to apply them with mixing at the affected part.

As a conventional applicator, there is the one configured as follows: from the tips of nozzles (spray heads) respectively connected to respective opening parts of two syringes respectively containing different types of liquids, respective liquids are ejected, and mixed (see, e.g., Patent Document 1, JP-A-2001-57979). The applicator described in this patent document 1 is configured as follows in order to mix the two liquids with reliability: respective liquids are ejected together with an aseptic gas, so that the respective liquids are ejected in an atomized form. The aseptic gas is fed from a cylinder filled with the aseptic gas connected to a nozzle via a tube. Further, in the cylinder, generally, a closable valve (cock) for controlling supply / stop of supply of the gas with respect to the nozzle is set. In the applicator described in the patent document 1, when the applicator is used, the valve is previously rendered in an opened state.

However, when the valve is previously rendered in an opened state, unfavourably, the aseptic gas continues being supplied to be involuntarily ejected from the nozzle irrespective of ejection / stop of ejection of the liquid. This causes the following disadvantages: only the aseptic gas continues hitting on the affected part; the aseptic gas is wasted; and other disadvantages. Further, there is another problem as follows: when the applicator is used with the valve rendered in a close state by mistake, respective liquids are ejected without having been atomized, so that the two liquids are applied in an insufficiently mixed state to the affected part.

### DISCLOSURE OF THE INVENTION

It is an object of the present invention to provide an applicator capable of carrying out supply / stop of supply of a gas with respect to a nozzle with ease and with reliability when a liquid is discharged / stopped being discharged from the nozzle.

In order to attain the foregoing object, the invention is:
an applicator, to be used with a first syringe and a second syringe respectively mounted therein, the first syringe and the second syringe, including: syringe outer tubes each having an opening part formed in a protruding manner at the distal end part; gaskets inserted in the syringe outer tubes; and pushers for moving and operating the gaskets along the longitudinal direction of the syringe outer tubes, and being filled with liquids in the spaces formed by the syringe outer tubes and the gaskets,
the applicator, which includes:
an applicator main body for arranging and fixing the first syringe and the second syringe;
a gas flow path being connected to a gas supply means for supplying a gas, and through which a gas from the gas supply means passes;
a nozzle for discharging therethrough the gas which has passed through the gas flow path, the liquid which has passed though the opening part of the first syringe, and the liquid which has passed though the opening part of the second syringe,
an operation part for pressing and operating the pusher of the first syringe and the pusher of the second syringe in the direction of the distal end; and
an opening and closing means provided in the operation part and for shutting off / opening the gas flow path,
wherein the opening and closing means operates so as to open the gas flow path in synchronization with the pressing operation by the operation part.

In accordance with such an aspect of the invention, it is possible to carry out supply / stop of supply of a gas with respect to a nozzle when a liquid is discharged / stopped being discharged from the nozzle with ease and with reliability.

Further, in the applicator of the invention, it is preferable that the opening and closing means is set midway in the gas flow path, and is formed of a closable valve mechanism.

As a result, it is possible to carry out supply / stop of supply of a gas with respect to a nozzle when a liquid is discharged / stopped being discharged from the nozzle with ease and with reliability.

Still further, in the applicator of the invention, it is preferable that the valve mechanism has a first connection part being connected to the upstream side of the gas flow path, and being in the shape of a tube, a second connection part being connected to the downstream side of the gas flow path, being in the shape of a tube, and being displaceable in a first posture in which it is identical in axis with the first connection part, and in a second posture in which the axis is tilted in the direction of operation of the operation part, and a valve part being closed when the second connection part is in the first posture, and being opened when the second connection part is in the second posture.

As a result, the valve mechanism can be opened / closed with reliability in synchronization with the pressing operation by the operation part. Accordingly, when the valve mechanism is in a closed state, it can shut off the flow of a gas from the gas supply means till the nozzle with reliability. When the valve mechanism is in an opened state, it can open (ensure) the flow of the gas with reliability.

Furthermore, in the applicator of the invention, it is preferable that the valve part has a sealing member formed of an elastic material and for air-tightly connecting the inner circumferential part of the first connection part and the outer circumferential part of the second connection part, a flange part provided with a diameter larger than the outer diameter of the second connection part on the upstream side of the gas flow path of the second connection part, and an urging part for urging the flange part to the side of the sealing member, and is configured such that the flange part is urged by the urging part to be in air-tight contact with the sealing member when the second connection part is in the first posture, and is displaced against the urging force of the urging part to cause a gap between it and the sealing member when the second connection part is in the second posture.

As a result, the valve mechanism can be opened / closed with reliability in synchronization with the pressing operation by the operation part. When the valve mechanism is in a closed state, it can shut off the flow of a gas from the gas supply means till the nozzle with reliability. When the valve mechanism is in an opened state, it can open (ensure) the flow of the gas with reliability.

Further, the applicator of the invention is preferably configured such that from the nozzle, the gas which has passed through the gas flow path is discharged roughly at the same time as or before the liquid to be ejected first of the two liquids of the liquid which has passed through the opening part of the first syringe and the liquid which has passed through the opening part of the second syringe.

As a result, it is possible to prevent only the liquid from being ejected and applied. Further, when a gas is discharged before each liquid, the two liquids are ejected in an atomized form respectively by the gas with reliability. As a result, these liquids are mixed with each other with reliability. Whereas, when the gas and each liquid are discharged roughly at the same time, it is possible to control wasteful ejection of the gas, i.e., waste of the gas. Further, it is possible to prevent only the gas from continuing being ejected.

Still further, in the applicator of the invention, it is preferable that the force for causing the gap between the sealing member and the flange part is set so as to be larger than the force for moving the pusher of the first syringe and the pusher of the second syringe in the direction of the distal end.

As a result, it is possible to prevent only the liquid from continuing being ejected and applied.

Further, the applicator of the invention preferably further includes an urging force adjusting means for adjusting the urging force of the urging part.

As a result, it is possible to set the optimal ejection timing of the nozzle.

Still further, in the applicator of the invention, it is preferable that the operation part has a connection part for connecting the proximal end parts of the pushers of the first syringe and the second syringe with each other, and a pressing part to be pressed by a user.

As a result, when the pressing part is pressed and operated, the pusher of the first syringe and the pusher of the second syringe can be moved integrally.

Furthermore, in the applicator of the invention, it is preferable that the applicator main body has a finger rest part on which a finger is rested, and with a plurality of fingers except for a thumb of one hand rested on the finger rest part, the thumb of the one hand is rested on the pressing part.

As a result, it is possible to grasp the applicator with stability and with reliability. Further, it is possible to carry out a pressing operation on the pressing part with smoothness and with reliability. Accordingly, the operability of the applicator is improved.

Whereas, in the applicator of the invention, it is preferable that the applicator main body has a first fitting part with which the opening parts of the first syringe and the second syringe are each fitted, and a second fitting part which is provided closer to the proximal end than the first fitting part, and with which the proximal end parts of the first syringe and the second syringe are each fitted.

As a result, when the first syringe and the second syringe are respectively mounted, these syringes can be fixed side by side with reliability.

Still further, in the applicator of the invention, it is preferable that the nozzle has a first discharge port for discharging the liquid of the first syringe therethrough, a first flow path for feeding the liquid which has flowed from the first syringe to the first discharge port, a second discharge port for discharging the liquid of the second syringe therethrough, a second flow path for feeding the liquid which has flowed from the second syringe to the second discharge port, gas discharge ports provided roughly concentrically at the outer circumferential parts of the first discharge port and the second discharge port, respectively, and for discharging therethrough the gas from the gas supply means, and a third flow path for feeding the gas supplied from the gas supply means to the gas discharge ports.

As a result, when the gas is discharged at high speed from each gas discharge port, into the gas discharged at high speed, the liquid discharged from the first discharge port and the liquid discharged from the second discharge port are caught (mixed). At this step, both the liquids are each ejected in an atomized form, and as a result, are mixed with reliability.

Still further, in the applicator of the invention, it is preferable that the fist syringe and the second syringe are filled with liquids different in liquid composition, respectively.

For example, when the applicator of the invention is used for administration of a biological tissue adhesive, one of the two liquids can be a liquid (solution or the like) containing thrombin, and the other can be a liquid (solution or the like) containing fibrinogen. Alternatively, for example, when the applicator of the invention is used for administration of an adhesion preventive material, one of the two liquids can be a liquid (solution or the like) containing carboxymethyl dextrin modified with a succinimidyl group, and the other can be a liquid (solution or the like) containing disodium hydrogenphosphate.

Whereas, with the applicator of the invention, it is preferable that the two liquids different in liquid composition are mixed with each other, thereby to serve as an adhesion preventive material of a biological tissue.

As a result, the two liquids gelate (solidity) upon mixing. The gelated two liquids can remain at the biological tissue (objective site) on which they have been applied with reliability. Further, at the objective site, they can exert a function as an adhesion preventive material at the objective site with reliability.

### BRIEF DESCRIPTION OF THE DRAWINGS

FIG. 1 is a perspective view showing a first embodiment of an applicator of the present invention;
FIG. 2 is a perspective view showing the first embodiment of the applicator of the invention;
FIG. 3 is a cross sectional view along line A-A of an opening and closing means (a view showing the state in which a gas flow path is shut off) in the applicator shown in FIG. 1;
FIG. 4 is a cross sectional view along line A-A of the opening and closing means (a view showing the state in which a gas flow path is opened) in the applicator shown in FIG. 1;
FIGS. 5A to 5E are longitudinal cross sectional views (views showing changes with time in the applied state) of the distal end part of a nozzle in the applicator shown in FIG. 1;
FIG. 6 is a longitudinal cross sectional view of the opening and closing means in an applicator (a second embodiment) of the invention; and
FIG. 7 is a partial longitudinal cross sectional view of a first syringe to be mounted in the applicator shown in FIG. 1 (the same also applies to a second syringe).

### BEST MODE FOR CARRYING OUT THE INVENTION

Below, an applicator of the present invention will be described in details based on preferred embodiments shown in the accompanying drawings.

### <First Embodiment>

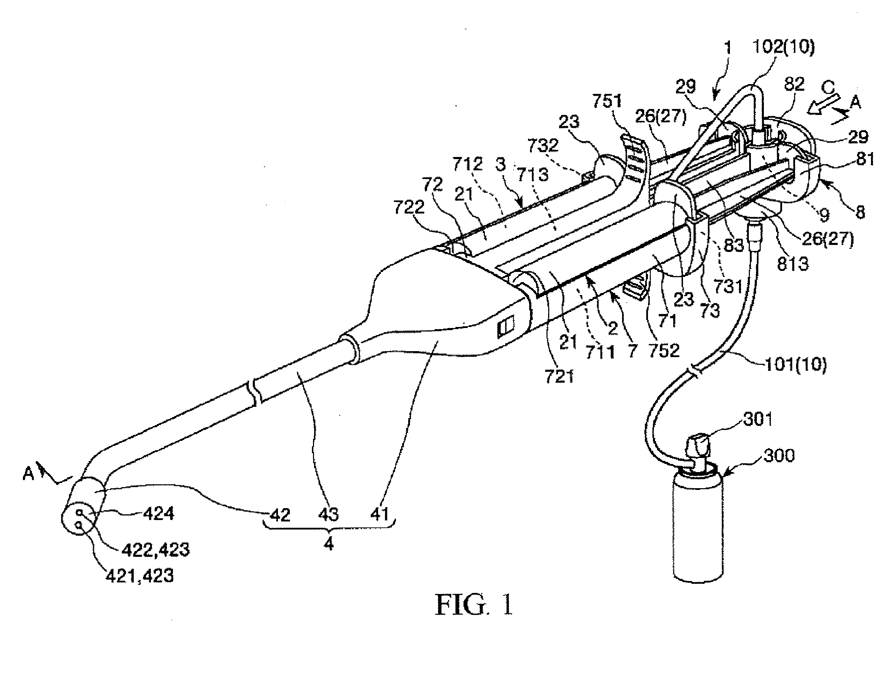

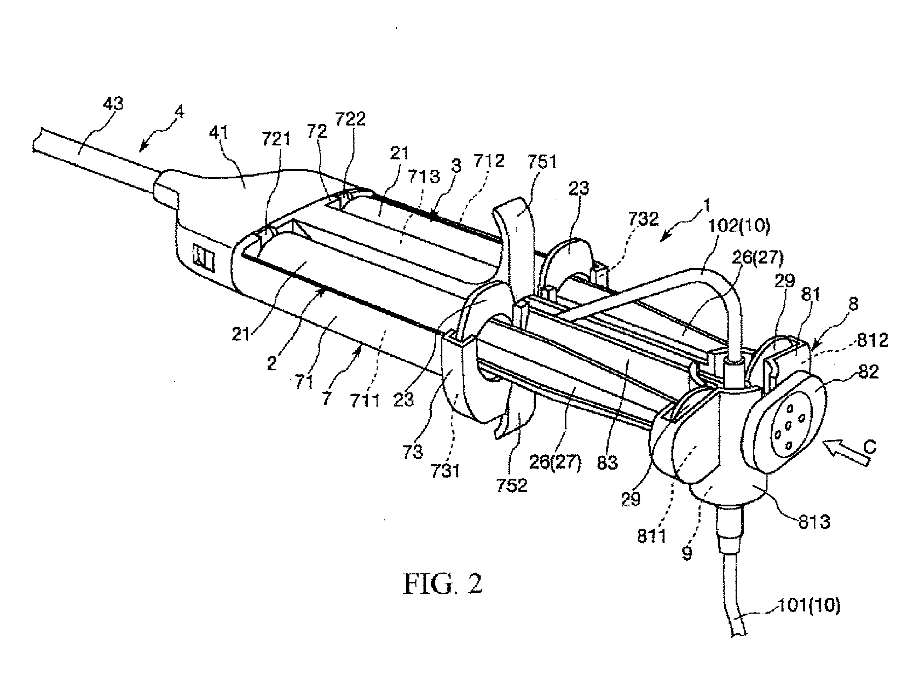

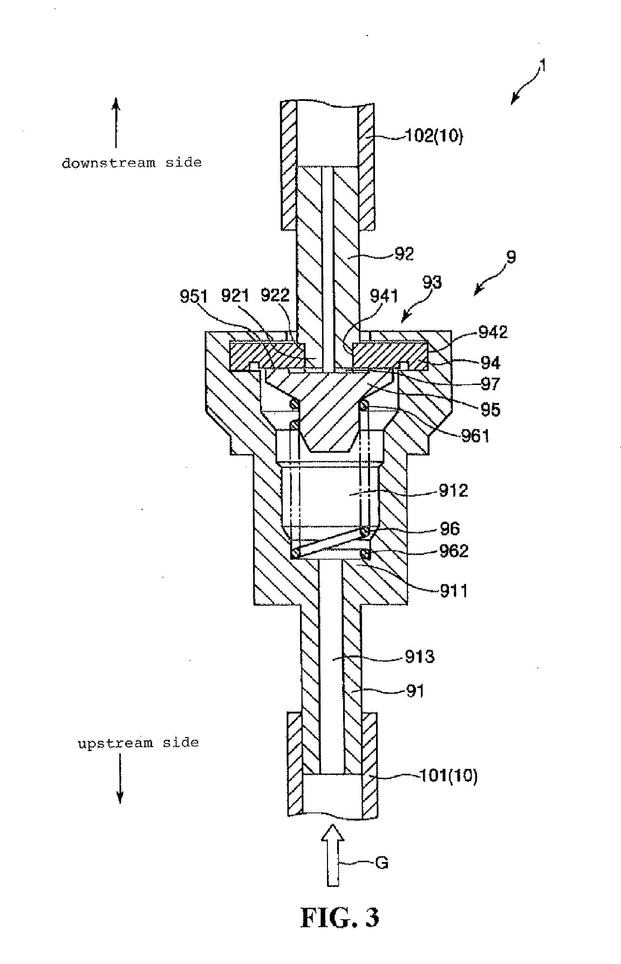

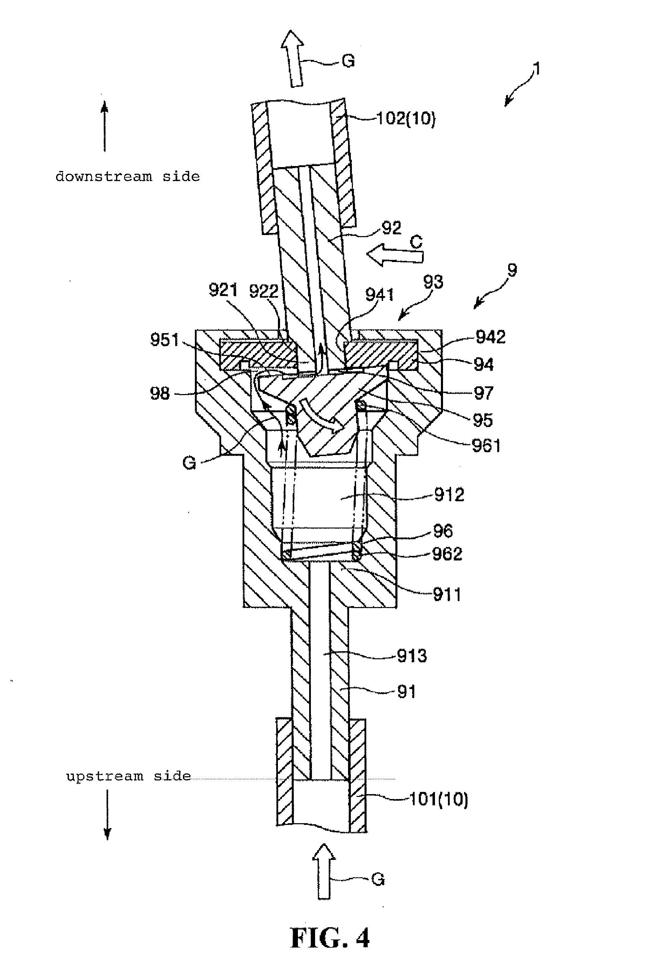

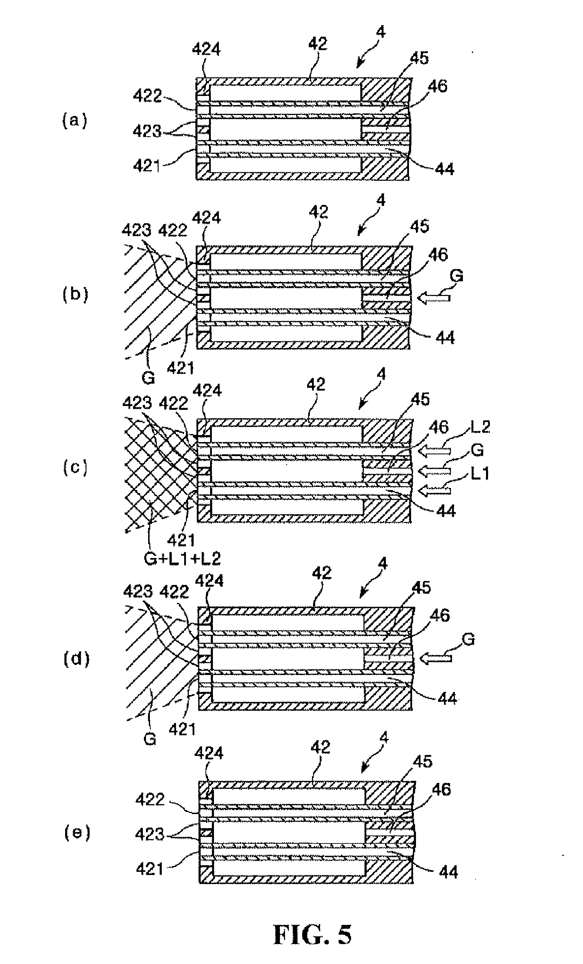

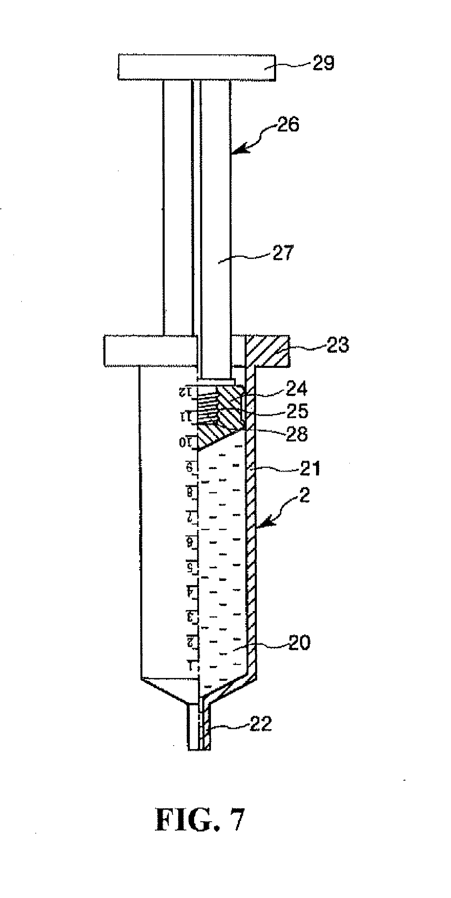

FIGS. 1 and 2 are respectively perspective views each showing a first embodiment of an applicator of the invention; FIGS. 3 and 4 are respectively cross sectional views each along line A-A of an opening and closing means (FIG. 3 showing the state in which a gas flow path is shut off, and FIG. 4 showing the state in which the gas flow path is opened) in the applicator shown in FIG. 1; FIGS. 5A to 5E are longitudinal cross sectional views (views showing changes with time in the applied state) of the distal end part of a nozzle in the applicator shown in FIG. 1; and FIG. 7 is a partial longitudinal cross sectional view of a first syringe to be mounted in the applicator shown in FIG. 1 (the same also applies to a second syringe). Incidentally, for convenience in description, the left hand side in FIGS. 1, 2 and 5A to 5E are each referred to as a "distil end"; and each right hand side, a "rear end (proximal end)". In FIG. 7, the lower side is referred to as a "distal end"; and the upper side, "a rear end". Further, in FIGS. 1 to 4, the upper side is referred to as a "top"; and the lower side, a "bottom".

As shown in FIGS. 1 and 2, an applicator 1 of the invention is used with a first syringe 2 and a second syringe 3 respectively mounted therein.

First, before describing the configuration of the applicator 1, the configurations of the first syringe 2 and the second syringe 3 will be describe. The first syringe 2 and the second syringe 3 are roughly the same in configuration, and hence, typically, the first syringe 2 will be described.

As shown in FIG. 7, the first syringe 2 in this embodiment includes an outer tube (syringe outer tube) 21, a gasket 24 slidable in the outer tube 21, and a pusher (plunger rod) 26 for moving and operating the gasket 24 along the longitudinal direction (axial direction) of the outer tube 21. The gasket 24 is connected to the distal end of the pusher 26.

The outer tube 21 is formed of a bottomed tubular member. At the central part of the bottom on the distal end side, a reduced diameter part (opening part) 22 reduced in diameter with respect to the body of the outer tube 21 is formed in a protruding manner integrally.

Around the periphery of the rear end of the outer tube 21, the flange 23 is integrally formed.
Whereas, on the outer circumferential surface of the outer tube 21, a scale indicative of the liquid amount is provided.

As the constituent materials of the outer tube 21, for example, mention may be made of various resins including polyvinyl chloride, polyethylene, polypropylene, cyclic polyolefin, polystyrene, poly-(4-methylpenetene-1), polycarbonate, acrylic resin, acrylnitrile-butadiene-styrene copolymer, polyesters such as polyethylene terephthalate and polyethylene naphthalate, butadiene-styrene copolymer, and polyamide (e.g., nylon 6, nylon 6·6, nylon 6·10, or nylon 12). Out of these, resins such as polypropylene, cyclic polyolefin, and polyesters are preferred in terms of ease of molding and low water vapor permeability. Incidentally, it is preferable that the constituent material of the outer tube 21 is substantially transparent in order to ensure the visibility of the inside.

In such an outer tube 21, the gasket 24 formed of an elastic material is stored (inserted). Around the periphery of the gasket 24, a plurality of (two in this embodiment) ring-like projections are formed around the entire circumference. The projections slide while being in close contact with the inner circumferential surface of the outer tube 21. As a result, the fluid tightness is held with more reliability, and the slidability can be improved.

Whereas, in the gasket 24, a hollow part 25 opening toward the rear end side is formed. In the hollow part 25, a head part 28 of the pusher 26 described later is screwed (engaged). In the inner surface of the hollow part 25, a female screw is formed.

The constituent material of the gasket 24 has no particular restriction. Examples thereof may include elastic materials including various rubber materials such as natural rubber, butyl rubber, isoprene rubber, butadiene rubber, styrene-butadiene rubber, and silicone rubber, various thermoplastic elastomers of polyurethane type, polyester type, polyamide type, olefin type, styrene type, and other types, or mixtures thereof.

The pusher 26 has a rod-like main body part 27 cross-shaped in cross section.
On the distal end side of the main body part 27, a head part (connecting part) 28 to be inserted into the hollow part 25 of the gasket 24, and connected to the gasket 24 is formed. Around the periphery of the head part 28, a male screw capable of screwing together with the female screw in the inner surface of the hollow part 25 is formed. By screwing the male screw together with the female screw, the gasket 24 and the pusher 26 are connected. Incidentally, not limited to connection by screwing together, the gasket 24 and the pusher 26 may be configured to be connected by, for example, concavo-convex fitting, may be configured to be attached by adhesion, fusion, or the like, or may be configured to be integrally formed.

Whereas, on the rear end side of the main body part 27, a disk-like flange 29 is formed.
Further, as the constituent materials of the pusher 26, there can be used the same ones as those exemplified as the constituent materials of the outer tube 21 described above.

The first syringe 2 is filled with a first liquid L1 described later in a space (liquid storage space) 20 surrounded by the outer tube 21 and the gasket 24 before being mounted in the applicator 1.

As with the first syringe 2, the second syringe 3 is also formed of the outer tube 21, the gasket 24 capable of sliding in the outer tube 21, and the pusher 26 for moving and operating the gasket 24. The space 20 is filled with a second liquid L2. The configuration of each part is the same, and hence a description thereon is omitted.

The first liquid L1 to be filled in the first syringe 2 and the second liquid L2 to be filled in the second syringe 3 are different in composition (components) thereof from each other.

In the invention, the first liquid L1 and the second liquid L2 are appropriately selected according to the use of the applicator 1, the intended purpose, the case, and the like. For example, when they are used for administration of a biological tissue adhesive, one of the first liquid L1 and the second liquid L2 can be a liquid (solution or the like) containing thrombin, and the other can be a liquid (solution or the like) containing fibrinogen.

Alternatively, when they are used for administration of an adhesion preventive material, one of the first liquid L1 and the second liquid L2 can be a liquid (solution or the like) containing carboxymethyl dextrin modified with a succinimidyl group, and the other can be a liquid (solution or the like) containing disodium hydrogenphosphate.

Such a combination of the first liquid L1 and the second liquid L2 gelate (solidify) upon mixing. The gelation enables, for example, the mixture of the first liquid L1 and the second liquid L2 (which is hereinafter referred to as a "mixture") to remain at the biological tissue (objective site) on which it has been applied with reliability. Further, the mixture remains at the objective site with reliability. Therefore, it can exert a function as a biological tissue adhesive or an adhesion preventive material at the objective site with reliability.

Incidentally, it is needless to say that the types and the combinations of the first liquid L1 and the second liquid L2 are not limited to the foregoing ones.

The applicator 1 in which the first syringe 2 filled with the first liquid L1 and the second syringe 3 filled with the second liquid L2 are mounted has an applicator main body 7, a nozzle 4, an operation part 8, an opening and closing means (valve mechanism) 9, and a tube 10 connected to a cylinder (gas supply means) 300 (see FIG. 1).

Before describing respective parts forming the applicator 1, the cylinder 300 will be described.
The cylinder 300 includes a high pressure (compressed) aseptic gas G (which is hereinafter simply referred as a "gas G") filled in the internal space. Thus, it can supply the gas G to the applicator 1 (nozzle 4) . To the cylinder 300, a closable valve (cock) for controlling supply / stop of supply of the gas G with respect to the applicator 1 is set. When the applicator 1 is used, the valve 301 is rendered in an open state.

As shown in FIGS. 1 and 2, the applicator main body 7 is for fixing the first syringe 2 and the second syringe 3 side by side (in parallel). The applicator main body 7 has a base 71, a front plate (first fitting part) 72 provided at the distal end of the base 71, a rear plate (second fitting part) 73 provided at the rear end of the base 71, and finger rest parts 751 and 752 provided in the vicinity of the rear plate 73 of the base 71.

In the base 71, at the upper part, concave parts 711 and 712 formed roughly in a semi-circular arc in cross section are provided in parallel. In the concave part 711, the outer tube 21 of the first syringe 2 is stored. In the concave part 712, the outer tube 21 of the second syringe 3 is stored.

At the distal end of the base 71, the front plate 72 is provided. In the front plate 72, grooves 721 and 722 are formed at the positions respectively corresponding to the concave parts 711 and 712. When the first syringe 2 and the second syringe 3 are mounted, the reduced diameter part 22 of the first syringe 2 is inserted into the groove 721, and the reduced diameter part 22 of the second syringe 3 is inserted into the groove 722.

At the rear end of the base 71, the rear plate 73 is provided. In the rear plate 73, the concave parts 731 and 732 are formed at the positions respectively corresponding to the concave parts 711 and 712. When the first syringe 2 and the second syringe 3 are mounted, the flange 23 (the proximal end part) of the first syringe 2 is fitted (inserted) into the concave part 731, and the flange 23 (proximal end part) of the second syringe 3 is fitted into the concave part 732.

Thus, in the applicator main body 7, each reduced diameter part 22 is fitted into the front plate 72, and each flange 23 is fitted into the rear plate 73. As a result, it is possible to fix the first syringe 2 and the second syringe 3 in parallel with reliability.

In the vicinity of the rear plate 73 of the base 71, the finger rest parts 751 and 752 are provided. On the finger rest parts 751 and 752, user's fingers can be rested, respectively, for use of the applicator 1. The finger rest part 751 is formed of an upwardly protruding plate piece, and the finger rest part 752 is formed of a downwardly protruding plate piece. Further, respective finger rest parts 751 and 752 are configured such that the sides facing the distal end direction each form a circular arc (curved concave shape).

The applicator main body 7 may be configured such that respective parts forming the applicator main body 7 are integrally formed, or may be configured such that respective parts are respectively formed of separate bodies, and these are bonded together.

The constituent material of the applicator main body 7 has no particular restriction. For example, various metal materials, various plastics, and the like may be used alone, or in combination thereof. When such a material is used, the applicator main body 7 can be manufactured with ease by, for example, injection molding.

On the rear end side of the applicator main body 7, the operation part 8 is set movably in the longitudinal direction with respect to the applicator main body 7. The operation part 8 is a site for pressing and operating the pusher 26 of the first syringe 2 and the pusher 26 of the second syringe 3 in the direction of the distal end (in the direction of an arrow C in FIGS. 1, 2, and 4). The operation parts 8 has a connection part 81 for connecting the flanges 29 of the pushers 26 of the first syringe 2 and the second syringe 3, a pressing part 82 situated on the rear end side of the connection part 81, and a rail part 83 extending from the connection part 81 toward the direction of the distal end.

In the connection part 81, upwardly opening concave parts 811 and 812 are respectively provided. The concave part 811 is in a shape corresponding to the flange 29 of the pusher 26 of the first syringe 2, in which the flange 29 is fitted (see FIG. 2). Whereas, the concave part 812 is in a shape corresponding to the flange 29 of the pusher 26 of the second syringe 3, in which the flange 29 is fitted (see FIG. 2).

By the connection part 81 with such a configuration, it is possible to connect and fix the flanges 29 of the pushers 26 of the first syringe 2 and the second syringe 3 with reliability. As a result, it is possible to move these pushers 26 integrally in the direction of the arrow C.

Whereas, in the connection part 81, a tubular part 813 formed in a tube is provided between the concave part 811 and the concave part 812. The tubular part 813 is provided so that its axis is in parallel with the vertical direction in FIG. 1 (the same also applies to FIG. 2). Further, in the tubular part 813, most of the opening and closing means 9 is stored.

At the outer circumferential part of the tubular part 813 of the connection part 81, a long-shaped rail part 83 is formed in a manner protruding toward the direction of the distal end. The rail part 83 is provided at the base 71 of the applicator main body 7, and inserted into a long-shaped concave part 713. The pressing operation in the direction of the arrow C of the operation part 8 guides the rail part 83 to the concave part 713. As a result, it is possible to carry out the pressing operation smoothly.

On the rear end side of the tubular part 813 of the connection part 81, the plate-shaped pressing part 82 is set movably in the longitudinal direction of the applicator main body 7 with respect to the tubular part 813.

The pressing part 82 is a site to be pressed by a user when the applicator 1 is used, i.e., the mixture is applied onto the affected part or the like. When the applicator 1 is used, for example, an index finger can be rested on the finger rest part 751, a middle finger can be rested on the finger rest part 752, and a thumb can be rested on the pressing part 82. As a result, it is possible to grasp the applicator 1 with stability and with reliability. Further, it is possible to carry out the pressing operation of the operation part 8 (pressing part 82) with smoothness and with reliability. This results in an improvement of the operability of the applicator 1.

Whereas, the pressing part 82 is connected to a second connection part 92 of the opening and closing means described later.
The constituent material of the operation part 8 has no particular restriction. For example, the materials as cited in connection with the description on the applicator main body 7 can be used. When such a material is used, the operation part 8 can be manufactured with, for example, injection molding, with ease.

At the front plate 72 of the applicator main body 7, the nozzle 4 is set. The nozzle 4 is for discharging therethrough the gas G (gas) which has passed through the tube 10, the first liquid L1 which has passed through the reduced diameter part 22 of the first syringe 2, and the second liquid L2 which has passed through the reduced diameter part 22 of the second syringe 3 (see FIGS. 5A to 5E). As shown in FIG. 1, the nozzle 4 has a nozzle main body 41, a nozzle head 42 situated on the distal end side of the nozzle main body 41, and a connection part 43 for connecting the nozzle main body 41 and the nozzle head 42.

The nozzle main body 41 is in the shape of a block formed of, for example, a metal material or a resin material. To the nozzle main body 41, the reduced diameter part 22 of the first syringe 2 and the reduced diameter part 22 of the second syringe 3, and the tube 10 are fluid-tightly (air-tightly) fitted and connected.

As shown in FIGS. 1 and 5A to 5E, the nozzle head 42 is cylindrical in outer shape. The nozzle head 42 has, in a distal end wall part 424, a first discharge port 421 for discharging the first liquid L1 therethrough, a second discharge port 422 for discharging the second liquid L2 therethrough, and a pair of third discharge ports (gas discharge ports) 423 for discharging the gas G therethrough.
The first discharge port 421 and the second discharge port 422 are provided adjacent to each other. Further, in this embodiment, the first discharge port 421 and the second discharge port 422 are equal to each other in size. Respective third discharge ports 423 are provided around the outer circumferential parts of the first discharge port 421 and the second discharge port 422 roughly concentrically, respectively.

The constituent material of the nozzle head 42 has no particular restriction. For example, the same ones as the constituent material of the nozzle main body 41 can be used.

As shown in FIG. 1, the connection part 43 is a long-shaped one. It connects the distal end of the nozzle main body 41 and the proximal end of the nozzle head 42. The connection part 43 may be either the one formed of a hard material, or the one formed of a soft material, an elastic material, or the like, and having flexibility. Examples of the constituent material of the connection part 43 may include: various soft and hard resins including polyvinyl chloride, polyethylene, polypropylene, cyclic polyolefin, polystyrene, poly-(4-methylpentene-1), polycarbonate, acrylic resin, acrylnitrile-butadiene-styrene copolymer, polyesters such as polyethylene terephthalate and polyethylene naphthalate, butadiene-styrene copolymer, and polyamide (e.g., nylon 6, nylon 6·6, nylon 6·10, or nylon 12), various rubber materials such as natural rubber, butyl rubber, isoprene rubber, butadiene rubber, styrene-butadiene rubber, and silicone rubber, various thermoplastic elastomers of polyurethane type, polyester type, polyamide type, olefin type, styrene type, and other types, various metal materials such as stainless steel, aluminum, copper, or copper type alloys, and various ceramics such as various glasses, alumina, and silica.

Whereas, in the nozzle 4, a first flow path 44, a second flow path 45, and a third flow path 46 penetrating through the nozzle main body 41, the connection part 43, and the nozzle head 42 are formed along the axis (the longitudinal direction) of the connection part 43. The first flow path 44 communicates with the first discharge port 421. Thus, it can feed the first liquid L1 which has passed through the reduced diameter part 22 of the first syringe 2 into the first discharge port 421. The second flow path 45 communicates with the second discharge port 422. Thus, it can feed the second liquid L2 which has passed through the reduced diameter part 22 of the second syringe 2 into the second discharge port 422. The third flow path 46 communicates with the respective third discharge ports 423. Thus, it can feed the gas G which has been supplied from the cylinder 300 and has passed through the tube 10 to the respective third discharge ports 423.

With the nozzle 4 having such a configuration, the gas G is discharged at high speed from each third discharge port 423. Into the gas G discharged at high speed, the first liquid L1 discharged from the first discharge port 421 and the second liquid L2 discharged from the second discharge port 422 are caught (mixed). At this step, the first liquid L1 and the second liquid L2 are each discharged in an atomized form. As a result, the first liquid L1 and the second liquid L2 are mixed with reliability, and applied onto the affected part.

Whereas, to the nozzle 4, the tube 10 is connected. The tube 10 functions as a gas flow path through which the gas G supplied from the cylinder 300 passes. Further, the tube 10 is formed of a first tube 101 situated on the upstream side (on the side of the cylinder 300) via the opening and closing means 9, and a second tube 102 situated on the downstream side (on the side of the nozzle 4).

The constituent material of the tube 10 (the first tube 101 and the second tube 102) has no particular restriction. Examples thereof may include: polyolefins such as polyethylene, polypropylene, and methylene-vinyl acetate copolymer, polyvinyl chloride, polybutadiene, polyamide, and polyester. Out of these, particularly, polybutadiene is preferably used. Use of polybutadiene for the constituent material of the tube 10 results in an appropriate flexibility, and excellent chemical resistance, and chemical adsorption preventive property.

Incidentally, as described above, in the tubular part 813 of the operation part 8, the opening and closing means 9 is set. The opening and closing means 9 is for shutting off / opening the gas G from the cylinder 300 to the nozzle 4. The first tube 101 and the second tube 102 are shut off (see FIG. 3) / communicate with each other (see FIG. 4) by the operation of the opening and closing means 9 through the opening and closing means 9.

As shown in FIGS. 3 and 4, the opening and closing means 9 has a first connection part 91 connected to the first tube 101, a second connection part 92 connected to the second tube 102, and a closable valve part 93 stored in the first connection part 91.

The first connection part 91 is in the shape of a tube. In the bore of the first connection part 91, there is provided a storage part 912 situated on the downstream side and in which the valve part 93 is stored. Further, in the bore of the first connection part 91, there is provided a reduced diameter part 913 reduced in diameter than the inner diameter on the upstream side of the storage part 93. At the boundary between the reduced diameter part 913 and the storage part 912, there is formed a step part 911 showing a sharp change in inner diameter.

The second connection part 92 is in the shape of a tube. As described above, the second connection part 92 is connected to the pressing part 82 of the operation part 8. The second connection part 92 is supported at a bottom part 921 by a sealing member 94 of the valve part 93. Thus, it is set on the downstream side of the first connection part 91 via the sealing member 94. The second connection part 92 is displaceably set in a first posture in which it is identical in axis with the first connection part 91 (the state shown in FIG. 3) and in a second posture in which the axis is tilted in the direction of an arrow C (direction of operation) of the pressing part 82 (operation part 8) with the bottom part 921 as the fulcrum (the state shown in FIG. 4).

The valve part 93 has the sealing member 94 formed of an elastic material, a flange part 95 situated on the upstream side than the sealing member 94, and an urging part 96 for urging the flange part 95 to the side of the sealing member 94.

The sealing member 94 is in the shape of a ring. The sealing member 94 is in close contact with an outer circumferential part 922 of the bottom part 921 of the second connection part 92 at its inner circumferential part 941. Whereas, an outer circumferential part 942 of the sealing member 94 is in close contact with an inner circumferential part 914 of the storage part 912 of the first connection part 91. With such a sealing part 94, the first connection part 91 and the second connection part 92 are air-tightly connected via the sealing member 94.

The flange part 95 has an outer diameter larger than the outer diameter of the second connection part 92. The flange part 95 is disposed in an opposing relation with the bottom side of the second connection part 92 via a gap 97.

The urging part 96 is formed of a compressed spring in this embodiment. It is, in a compressed state, in contact with the flange part 95 at its upper edge 961, and in contact with the step part 911 of the first connection part 91 at its bottom part 962. This can urge the flange part 95 to the side of the sealing member 94 with reliability.

With the valve part 93 having such a configuration, when the second connection part 92 is in the first posture, i.e., when an external force is not applied to the second connection part 92, the flange part 95 is urged onto the urging part 96 to be air-tightly brought in close contact with the sealing member 94 (see FIG. 3). As a result, the valve part 93 is rendered in a closed state.

Whereas, when a pressing force in the direction of the arrow C by the pressing part 82 of the operation unit 8 acts on the second connection part 92, the second connection part 92 is displaced from the first posture to the second posture. At this step, the flange part 95 is displaced against the urging force of the urging part 96. As a result, a part (or the whole) of a peripheral part 951 of the flange part 95 is separated apart from the sealing member 94. This results in the formation of a gap 98 between it and the sealing member 94 (see FIG. 4). As a result, the gas G flows from the first connection part 91 into the second connection part 92 via the gap 98. Namely, the valve part 93 is rendered in an opened state.

With the opening and closing means 9 having the foregoing configuration, the valve part 93 can be opened / closed with reliability in synchronization with the pressing operation by the operation part 8. As a result, when the valve part 93 is in a closed state, the flow of the gas G from the cylinder 300 till the nozzle 4 can be shut off with reliability. When the valve part 93 is in an opened state, the flow of the gas G can be opened with reliability.

Incidentally, the constituent materials of the first connection part 91, the second connection part 92, the flange part 95, and the urging part 96 have no particular restriction. However, for example, various metal materials and various plastics may be used alone or in combination thereof.

Whereas, the constituent materials of the sealing member 94 have no particular restriction. However, for example, various rubber materials such as natural rubber, butyl rubber, isoprene rubber, butadiene rubber, styrene-butadiene rubber, and silicone rubber can be used.

Then, a description will be given to the operation state of the applicator 1 in a usable state, i.e., including the first syringe 2 filled with the first liquid L1 and the second syringe 3 filled with the second liquid L2 mounted therein, and connected to the cylinder 300.

The first syringe 2 and the second syringe 3 are filled with the first liquid L1 and the second liquid L2 each in an amount necessary (enough) to be applied onto the affected part, respectively. Whereas, for the cylinder 300, the valve 301 is in an opened state, which allows the gas G to be supplied to the applicator 1.

Whereas, for the applicator 1, a force for causing the gap 98 between the sealing member 94 and the flange part 95 against the force of the urging part 96 (urging force) for pressing the flange part 95 against the sealing member 94, i.e., the pressing force in the direction of the arrow C to tilt the second connection part 92 from the first posture to the second posture is set so as to be larger than the force to move the pusher 26 of the first syringe 2 and the pusher 26 of the second syringe 3 in the direction of the distal end. Such setting can be done in the following manner. For example, various conditions such as the spring constant of the urging part 96, the viscosity of each liquid, and the inner diameter of each outer tube 21 are appropriately set.

For such an applicator 1, first, for example, an index finger is rested on the finger rest part 751 of the applicator main body 7, a middle finger is rested on the finger rest part 752, and a thumb is rested on the pressing part 82 of the operation part 8. At this step, the gas G, the first liquid L1, and the second liquid L2 are not discharged from the nozzle 4 (see FIG. 5A).

Then, when the pressing part 82 is pressed and operated with a thumb in this state, first, the second connection part 92 is tilted. As a result, the gap 98 is caused between the sealing member 94 and the flange part95. Thus, the gas G passes through the gap 98 to flow (see FIG. 4). As a result, the gas G is ejected from each third discharge port 423 of the nozzle 4.

Further, the pressing part 82 is pressed. Then, the second connection part 92 is tilted to the limit, so that the pressing force from the thumb is transferred to the connection part 81 via the pressing part 82. As a result, the connection part 81 starts to move. Accordingly, the first liquid L1 is pushed out from the first syringe 2, and the second liquid L2 is also pushed out from the second syringe 3. The pushed first liquid L1 passes through the first flow path 44 of the nozzle 4 , and is ejected from the first discharge port 421 (see FIG. 5C). Whereas, the second liquid L2 passes through the second flow path 45 of the nozzle 4, and is ejected from the second discharge port 422 roughly as with the first liquid L1 (see FIG. 5C).

The ejected first liquid L1 and second liquid L2 are respectively atomized by the action of the gas G as described above, and mutually mixed to be applied onto the affected part.

Each pusher 26 is fully pushed by the pressing operation of the operation part 8. Namely, when the movement of each pusher 26 stops, the first liquid L1 and the second liquid L2 respectively stop being ejected (see FIG. 5D). At this step, the gas G is still being ejected.

Thereafter, when the thumb which has pressed the pressing part 82 is removed from the pressing part 82, the pressing force against the second connection part 92 is released. Thus, the second connection part 92 returns to the first posture. As a result, the gap 98 between the sealing member 94 and the flange part 95 disappears. Namely, the sealing member 94 and the entire circumference of the peripheral part 951 of the flange part 95 come in close contact with each other (see FIG. 3). As a result, the gas G stops being ejected from each third discharge port 423 (see FIG. 5E).

In this manner, with the applicator 1, it is possible to optimally set the timing of supply / stop of supply of the gas G with respect to the nozzle 4 when the first liquid L1 and the second liquid L2 are discharged / stopped being discharged from the nozzle 4. In other words, the applicator 1 is configured such that the gas G is discharged from the nozzle 4 before the first liquid L1 and the second liquid L2 with ease and with reliability.

This can prevent the first liquid L1 and the second liquid L2 from being applied onto the affected part without being mixed with the gas G. Further, by the previously ejected gas G, the first liquid L1 and the second liquid L2 are respectively ejected in an atomized form with reliability. As a result, these liquids are mixed with each other with reliability.

Further, the applicator 1 is configured such that the gas G is stopped being ejected later than the first liquid L1 and the second liquid L2. As a result, when each liquid stops being ejected, and thereby the liquid remains (is deposited) on each discharge port, the gas G can blow away these remaining liquids. As a result, it is possible to prevent the disadvantage (e.g. , clogging of each discharge port due to coagulation) caused by the reaction between the first liquid and the second liquid in the vicinity of each discharge port.

Alternatively, the applicator 1 may also be configured such that the gas G is discharged roughly at the same time as the first liquid L1 and the second liquid L2 from the nozzle 4. In this case, it is possible to control wasteful ejection of the gas G, i.e., waste of the gas G. Further, it is possible to prevent only the gas G from continuing being ejected onto the affected part.

### <Second Embodiment>

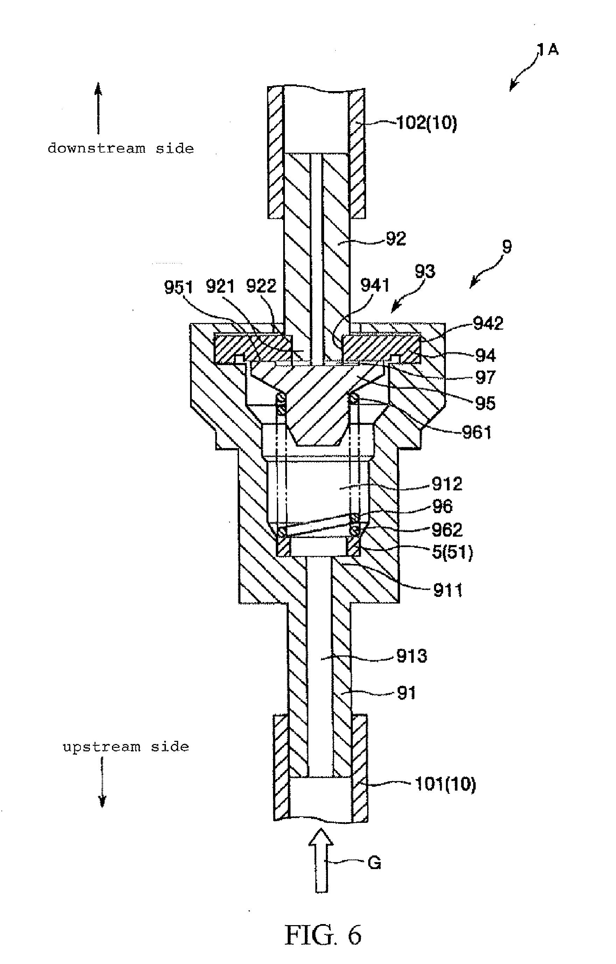

FIG. 6 is a longitudinal cross sectional view of the opening and closing means in an applicator (a second embodiment) of the invention.

Below, the second embodiment of the applicator of the invention will be described by reference to this drawing. However, a description will be given centering on the differences from the foregoing embodiment, and a description of the same items will be omitted.

This embodiment is the same as the first embodiment except that the applicator further has an urging force adjusting means.

The applicator 1A shown in FIG. 6 further includes an urging force adjusting means 5 for adjusting the urging force of the urging part 96. The urging force adjusting means 5 has no particular restriction. However, in this embodiment, it is formed of a ring member (spacer) 51 in the shape of a ring.

The ring member 51 has its outer diameter roughly the same as the outer diameter of the urging part 96. The ring member 51 is stored in the storage part 912 of the first connection part 91, and it is situated between the bottom part 962 of the urging part 96 and the step part 911. As a result, it is possible to compress the urging part 96 than the urging part 96 of the first embodiment. Accordingly, it is possible to adjust the urging force, i.e., to increase the urging force.

Incidentally, the force for moving the pusher 26 of the first syringe 2 and the pusher 26 of the second syringe 3 in the direction of the distal end (which is hereinafter referred to as a "moving force") varies according to, for example, the viscosities of the first liquid L1 and the second liquid L2, and the inner diameter of each outer tube 21.

For example, when the first liquid L1 to be filled in the first syringe 2 is the one having a relatively large viscosity, the moving force may be larger than the pressing force in the direction of the arrow C for tilting the second connection part 92 from the first posture to the second posture. For this reason, even when the pressing part 82 is pressed, the valve part 93 is rendered in an opened state. Thus, only the gas G continues being ejected, which makes it difficult for each pusher 26 to be pressed. Accordingly, the fist liquid L1 and the second liquid L2 may not be pressed out (may not be ejected).

Whereas, when the first liquid L1 to be filled in the first syringe 2 is the one having a relatively small viscosity, the moving force may be extremely smaller than the pressing force in the direction of the arrow C for tilting the second connection part 92 from the first posture to the second posture. For this reason, even when the pressing part 82 is pressed, first, each pusher 26 is pressed. Thus, only the first liquid L1 and the second liquid L2 are ejected. Thus, the valve part 93 is kept in a closed state, so that the gas G may not be ejected.

However, the applicator 1A has the urging force adjusting means 5. As a result, for example, as described above, even when the first liquid L1 is the one having a relatively large viscosity, by increasing the urging force of the urging part 96, the pressing force in the direction of the arrow C for tilting the second connection part 92 from the first posture to the second posture can be set so as to be larger than the moving force with reliability. As a result, from the nozzle 4, the gas G is ejected before the first liquid and the second liquid. Namely, it is possible to set the optimum ejection timing of the nozzle 4. For example, it is possible to blow out the first liquid L1 and the second liquid L2 after 0.2 second from ejection of the gas G.

Thus, with the applicator 1, it is possible to overcome the disadvantage due to changes in moving force described above.

Incidentally, the constituent materials of the ring member 51 have no particular restriction. However, for example, various metal materials and various plastics may be used alone, or in combination thereof.

Whereas, the urging force adjusting means 5 is not limited to the ring member 51. Examples thereof may include the following.

The portion defining the storage part 912 of the first connection part 91 is formed of a first member situated on the upstream side, and a second member situated on the downstream side than the first member. These members are bonded by screwing together. This enables the first member and the second member to move closer to each other, or away from each other. Accordingly, it is possible to adjust the distance between the flange part and the step part. As a result, the equal effects as with the ring member 51 are exerted.

Up to this point, the applicator of the invention were described by way of the embodiments shown. However, the present invention is not limited thereto. Each part forming the applicator can be replaced with the one having a given configuration capable of exerting the same function. Further, a given structure may be added thereto.

Further, the applicator is configured such that the first liquid which has passed through the reduced diameter part of the first syringe and the second liquid which has passed through the reduced diameter part of the second syringe are ejected from the nozzle roughly at the same time. However, the present invention is not limited thereto. For example, it may also be configured such that one of these two liquids is ejected before the other.

### INDUSTRIAL AVAILABILITY

An applicator of the present invention is used with a first syringe and a second syringe respectively mounted therein. The first syringe and the second syringe include:
syringe outer tubes each having an opening part formed in a protruding manner at the distal end part; gaskets inserted in the syringe outer tubes; and pushers for moving and operating the gaskets along the longitudinal direction of the syringe outer tubes, and are filled with liquids in the spaces formed by the syringe outer tubes and the gaskets. The applicator includes: an applicator main body for arranging and fixing the first syringe and the second syringe; a gas flow path being connected to a gas supply means for supplying a gas, and through which a gas from the gas supply means passes; a nozzle for discharging therethrough the gas which has passed through the gas flow path, the liquid which has passed though the opening part of the first syringe, and the liquid which has passed though the opening part of the second syringe; an operation part for pressing and operating the pusher of the first syringe and the pusher of the second syringe in the direction of the distal end; and an opening and closing means provided in the operation part and for shutting off / opening the gas flow path. The opening and closing means operates so as to open the gas flow path in synchronization with the pressing operation by the operation part. For this reason, it is possible to carry out supply / stop of supply of a gas with respect to a nozzle with ease and with reliability when a liquid is discharged / stopped being discharged from the nozzle. Therefore, the applicator of the invention has industrial availability.

## Claims

1. An applicator, to be used with a first syringe and a second syringe respectively mounted therein, the first syringe and the second syringe, comprising: syringe outer tubes each having an opening part formed in a protruding manner at the distal end part; gaskets inserted in the syringe outer tubes; and pushers for moving and operating the gaskets along the longitudinal direction of the syringe outer tubes, and being filled with liquids in the spaces formed by the syringe outer tubes and the gaskets,
the applicator, comprising:
an applicator main body for arranging and fixing the first syringe and the second syringe;
a gas flow path being connected to a gas supply means for supplying a gas, and through which a gas from the gas supply means passes;
a nozzle for discharging therethrough the gas which has passed through the gas flow path, the liquid which has passed though the opening part of the first syringe, and the liquid which has passed though the opening part of the second syringe,
an operation part for pressing and operating the pusher of the first syringe and the pusher of the second syringe in the direction of the distal end; and
an opening and closing means provided in the operation part and for shutting off / opening the gas flow path,
wherein the opening and closing means operates so as to open the gas flow path in synchronization with the pressing operation by the operation part.

2. The applicator according to claim 1, wherein the opening and closing means is set midway in the gas flow path, and is formed of a closable valve mechanism.

3. The applicator according to claim 2, wherein the valve mechanism has a first connection part being connected to the upstream side of the gas flow path, and being in the shape of a tube, a second connection part being connected to the downstream side of the gas flow path, being in the shape of a tube, and being displaceable in a first posture in which it is identical in axis with the first connection part, and in a second posture in which the axis is tilted in the direction of operation of the operation part, and a valve part being closed when the second connection part is in the first posture, and being opened when the second connection part is in the second posture.

4. The applicator according to claim 3, wherein the valve part has a sealing member formed of an elastic material and for air-tightly connecting the inner circumferential part of the first connection part and the outer circumferential part of the second connection part, a flange part provided with a diameter larger than the outer diameter of the second connection part on the upstream side of the gas flow path of the second connection part, and an urging part for urging the flange part to the side of the sealing member, and is configured such that the flange part is urged by the urging part to be in air-tight contact with the sealing member when the second connection part is in the first posture, and is displaced against the urging force of the urging part to cause a gap between it and the sealing member when the second connection part is in the second posture.

5. The applicator according to claim 4, configured such that from the nozzle, the gas which has passed through the gas flow path is discharged roughly at the same time as or before the liquid to be ejected first of the two liquids of the liquid which has passed through the opening part of the first syringe and the liquid which has passed through the opening part of the second syringe.

6. The applicator according to claim 5, wherein the force for causing the gap between the sealing member and the flange part is set so as to be larger than the force for moving the pusher of the first syringe and the pusher of the second syringe in the direction of the distal end.

7. The applicator according to claim 6, further comprising an urging force adjusting means for adjusting the urging force of the urging part.

8. The applicator according to claim 1 or 2, wherein the operation part has a connection part for connecting the proximal end parts of the pushers of the first syringe and the second syringe with each other, and a pressing part to be pressed by a user.

9. The applicator according to claim 8, wherein the applicator main body has a finger rest part on which a finger is rested, and with a plurality of fingers except for a thumb of one hand rested on the finger rest part, the thumb of the one hand is rested on the pressing part.

10. The applicator according to claim 1 or 2, wherein the applicator main body has a first fitting part with which the opening parts of the first syringe and the second syringe are each fitted, and a second fitting part which is provided closer to the proximal end than the first fitting part, and with which the proximal end parts of the first syringe and the second syringe are each fitted.

11. The applicator according to claim 1 or 2, wherein the nozzle has a first discharge port for discharging the liquid of the first syringe therethrough, a first flow path for feeding the liquid which has flowed from the first syringe to the first discharge port, a second discharge port for discharging the liquid of the second syringe therethrough, a second flow path for feeding the liquid which has flowed from the second syringe to the second discharge port, gas discharge ports provided roughly concentrically at the outer circumferential parts of the first discharge port and the second discharge port, respectively, and for discharging therethrough the gas from the gas supply means, and a third flow path for feeding the gas supplied from the gas supply means to the gas discharge ports.

12. The applicator according to claim 1 or 2, wherein the fist syringe and the second syringe are filled with liquids different in liquid composition, respectively.

13. The applicator according to claim 12, wherein the two liquids different in liquid composition are mixed with each other, thereby to serve as an adhesion preventive material of a biological tissue.
